# EUROPEAN PATENT APPLICATION

(11) **EP 3 098 333 A1**
(43) Date of publication of application: **30.11.2016**
(21) Application number: 14880167.3
(22) Date of filing: 21.01.2014
(51) Int. Cl.: C25D 3/12, A61L 31/08, B82Y 30/00, C22C 19/03

(54) **ELECTROLYTIC BATH FOR PRODUCING ANTIBACTERIAL METAL COATINGS CONTAINING NICKEL, PHOSPHORUS AND NANOPARTICLES OF AN ANTIBACTERIAL METAL (NI-P-MANP'S)**

(71) Applicant: Centro de Investigación y Desarrollo Tecnológico en Electromecanica S.C., 76703 Pedro Escobedo, Querétaro (MX)
(72) Inventor: TREJO-CÓRDOVA, Gabriel, 76703 Pedro Escobedo, Querétaro (MX); DÁVALOS-BENÍTEZ, Celina Elizabeth, 76703 Pedro Escobedo, Querétaro (MX); MEAS-VONG, Yunny, 76703 Pedro Escobedo, Querétaro (MX); ORTEGA-BORGES, Raúl Martín, 76703 Pedro Escobedo, Querétaro (MX); RÍOS-ÁLVAREZ, Claudia, 76703 Pedro Escobedo, Querétaro (MX)
(74) Representative: Awapatent AB
(86) International application number: PCT/IB2014/000057
(87) International publication number: WO 2015/110851

(57) **Abstract**

The present invention proposes the use of an electrolytic bath to electroplate metal composites of nickel-phospohrous-metal nano-particles having antibacterial ability, which inhibits bacteria growing, such as Escherichia coli and Staphylococcus aureus, at least on 99% of its surface.

The method of formulating an electrolytic bath allowing to obtain antibacterial coatings includes the following steps: a) adding p3+ ions to an electrolytic bath containing dissolved Ni salts, b) adding to the electrolytic bath metal nano-particles having antibacterial ability suspended in a cationic surfactant, c) electroplating the antibacterial composite metal of Ni-P-metal by applying a direct current density. The occlusion of metal nano-particles having antibacterial ability in the coating matrix provides it with antibacterial features.

## Description

### FIELD OF THE INVENTION

The present invention relates to nickel (Ni) coatings containing occluded and homogeneously dispersed antibacterial metal agents in the whole coating, more specifically, to an electrolytic bath composition to obtain an antibacterial metal coating made of nickel-phosphorus-antibacterial metal nano-particles (Ni-P-MANPs). Metal coatings include decorative electroplated coatings for bathrooms, kitchen accessories, handrails, shopping charts, coins, doorknobs and other highly lustrous products wherein the antibacterial protection is necessary.

### OBJECTS OF THE INVENTION

The main object of the present invention is to provide an electrolytic bath which allows to obtain, by metal electroplating, Ni-P-MANPs coatings whose antibacterial metal nano-particles are occluded and homogeneously dispersed in the whole coating.

The second object of the present invention is to provide antibacterial metal coatings (Ni-P-MANPs) which prevent and inhibit bacteria growing, and/or which eliminate both bacteria types: Gram-negative, such as Escherichia coli, and Gram positive, such as Staphylococcus aureus, at least in 99% of its surface.

### BACKGROUND OF THE INVENTION

As a contribution to the solution of the severe health problems caused by the bacteria transmission among the population, currently, many finished products include antimicrobial agents in their composition. Nowadays, antimicrobial agents can be found in various consumer products, for example: soaps, dental cleaning products, deodorants, first aid products, kitchen items, clothing, paintings, washing machines, vacuum cleaners, etc. In the above examples, the antimicrobial agents are easily incorporated into the product since they are mixed in the formulations to manufacture the product.

In the same regard, presently the industry now requires antimicrobial coatings in many products with a metallic finishing, since many diseases can be spread from one person to another simply because both individuals contact the same metal product. The metal product can be: a door handle, the toilet handle, a waste container, shopping charts, public transportation handrails, cooking utensils, coins, doorknobs and other products with which human beings have contact. In the last years, regarding coatings, the trend has been the development of metal and polymeric coatings with antibacterial properties. In this regard, it is known that antibacterial agents can be added to various metals surface such as: stainless steel sheets, Chromium coatings or Zinc coatings, by using the thermal spraying technique, as described in Patent documents WO 2010069104A1, US 2012/0225312A1 and WO 2012122666A1. However, the thermal spraying process has the main disadvantage of its high cost, added to the fact that some of the metal characteristics, such as brightness and adherence, are altered by the antimicrobial film as formed.

Thermostable resin compositions that contain antimicrobial agents have also been developed to be used as coating materials for various metals, such as: iron, aluminum, copper and stainless steel, as described in Patent documents WO 2013052683A2, WO 2012158702A2, WO 2003043745A1 and WO 2013033802A1. However, these resin compositions include particulate materials, such as zeolites and oxides which can be undesired materials on the articles surface, for example, functional or decorative items, which have high aesthetic requirements.

In Patent document WO 1999025898A1, a simple process for forming antimicrobial films is used. In this process, from a solution having the antimicrobial components, which may be organic or inorganic, a thin film is applied to the metal surface and is further pressurized, without heating, in order to form the antibacterial coating.

Another process for forming antibacterial coatings is electroplating: In Patent document WO 2009120784A2 the electroplating is used to make antibacterial coatings from antibacterial organic agents dispersed on the coating surface. Likewise, in Patent documents EP 2438216A1 and EP 2522377A1, the electroplating process is used to form amorphous coatings from cobalt (Co) having antibacterial properties. In Patent document WO 2012135107A2, a silver (Ag) film is electroplated over stainless steel to form materials which can be used as implants. In this regard, metals such as silver and copper or their oxides, are considered as antimicrobial agents and they are known as antibacterial metals. Antibacterial metals are those which metal ions have an antibacterial effect and which are preferably biocompatible. Preferred biocompatible antibacterial metals include: Silver (Ag), Gold (Au), Copper (Cu), Platinum (Au), Palladium (Pd) and Iridium (Ir) (noble metals).

In Patent document WO 2004101014A2, silver oxidized species are chemically deposited over a surface, in order to form antibacterial coatings having an application in the medical material development. In addition, in Patent document WO 2007097790A1 silver oxides are deposited, such as: AgO and Ag2O, over polymeric surfaces, using the ion plasma deposition technique (IPD).

Currently, the largest unexploited market for antimicrobial protection is the highly decorative market and the galvanized finishing, including, but not limited to, bathroom accessories, iron fittings for doors, shopping charts, public transportation handrails, kitchen accessories such as: stove grills, refrigerator trays, ovens grills, pans, etc. These articles are usually coated with nickel, a zinc-nickel alloy or with chrome.

In the present invention, the electroplating process was used for forming metal composite coatings having an antibacterial metal occluded therein, such as silver or copper, homogeneously dispersed throughout the coating. Unlike other processes disclosed in the above-mentioned patents, which only form a single film of the antibacterial agent on the coating surface, in the present invention the antibacterial agent, namely an antibacterial metal as those referenced above, is homogeneously occluded in the metal matrix, thus forming part of the metal coating. The metal composite coating obtained by the electroplating process meets the high aesthetic standards required for decorative finishings including, but not limited to; brightness, high resistance to corrosion, high hardness. As well as the ability to prevent and to inhibit bacteria growth, and/or to eliminate both bacteria types: Gram-negative, such as Escherichia coli, and Gram positive, such as Staphylococcus aureus, at least in 99% of its surface.

In a preferred embodiment, the occluded antibacterial agents are nano-particles of an antibacterial metal, preferably silver or copper, and which disperse evenly throughout the whole thickness of the metal composite coating.

### TECHNICAL PROBLEM TO BE SOLVED

The processes currently known as thermal spraying or resin incorporation change the surface properties such as: brightness, wear resistance, adhesion. These processes do not incorporate metal nano-particles with antibacterial effects in a homogeneous way to the coating composition, on the contrary, they only form an antibacterial film on the surface.

### BRIEF DESCRIPTION OF THE INVENTION

The present invention consists of an electrolytic bath for electroplating a metal composite of Nickel-phosphorus-metal antibacterial nano-particles (Ni-P-MANPs), comprising salts such as Ni2+ ion sulfamates to be deposited, a pH buffering agent, a phosphorus-containing acid and which allows the formation of a nickel-phosphorus (Ni-P) alloy, and which increases the phosphorus concentration in the metal composite, moreover, it also contains antibacterial metal nano-particles (MANPs), with an average size between 10 and 100 nanometers (nm) and which may be silver (Ag) or copper (Cu) nano-particles, the occlusion of the MANPs in the metal matrix coating provides antibacterial properties to the metal composite coating, in addition, it contains a surfactant which allows keeping stable the MANPs in the suspension, and it makes easier their occlusion in the metal matrix composite during the metal ion electroplating. The composite coatings thus obtained from the electrolytic bath are able to prevent, inhibit and/or eliminate both bacteria types: Gram-negative, such as Escherichia coli, and Gram positive, such as Staphylococcus aureus, at least in 99% of its surface.

The present invention which consists of an electrolytic bath which allows to obtain, by metal electroplating, composite coatings of Ni-P-MANPs, was developed based on the following considerations: the metal ion salts to be deposited, which may be sulfamates, have the function of providing Ni2+ ions. Also, a phosphorus (P3+) based acid has the main function of forming the Ni-P alloy and to increase the phosphorus concentration in the composition of the Ni-P metal composite coating. Furthermore, the occlusion of the antibacterial metal nano-particles in the coating metal matrix provides the antibacterial features. Importantly, a cationic surfactant is used, which may be cetyl trimethyl ammonium hydrogensulfate, decyl trymethyl ammonium bromide, or sodium dodecyl sulfate, which stabilizes the MANPs in the suspension and provides them with a positive charge, which favors the occlusion of the nano-particles in the metal matrix during the electroplating process, producing metal composite coatings being homogeneous in composition.

The composite coatings obtained from the electrolytic bath, have a content between 1 and 4 mg/cm3 MANPs in the metal matrix, a phosphorus content between 25 and 70% and a nickel content between 30 and 70 %, depending on the conditions of the electroplating.

The assessment of the antibacterial ability of the coatings with regard to the Staphylococcus aureus and Escherichia coli bacterias was carried out according to the Mexican Official Standard NOM-109-SSA1-1994. The results showed that the Ni-P-MANPs coatings inhibit the growing of Escherichia coli bacteria between 98 and 100% and for the Staphylococcus aureus bacteria, between 71 and 100% depending on the concentration of silver nano-particles (AgNP's) in the coating.

### BRIEF DESCRIPTION OF THE DRAWINGS

The appended figures are explained as follows:
Fig. 1. Composition analysis of the Ni-Ag coating, carried out on a cross-sectioned sample. The analysis was performed by X-ray dispersing energy coupled to a scanning electron microscopy.

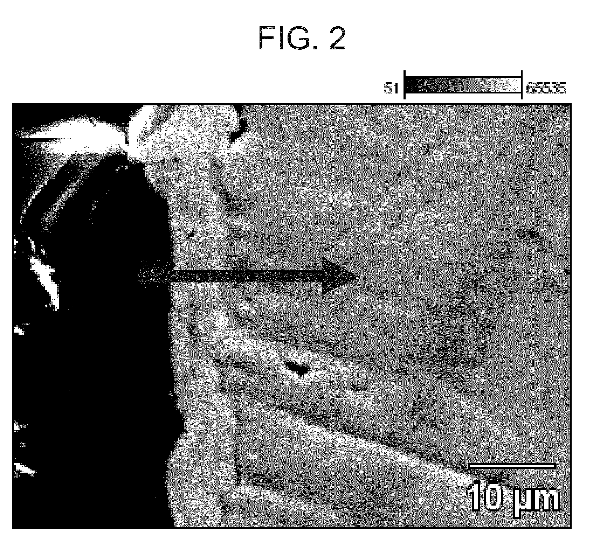

Fig 2. Scanning electronic microscopy analysis on a cross-sectioned sample.

### DETAILED DESCRIPTION OF THE INVENTION

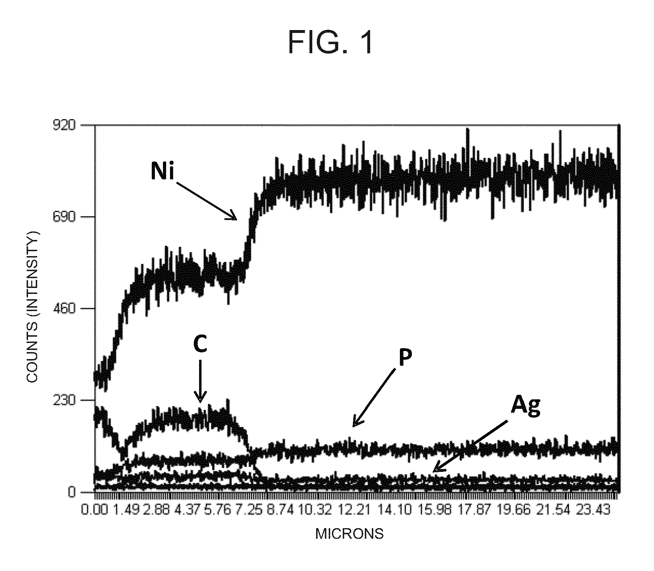

In the present invention an electrolytic bath is used which contains:
- Ni2+ ions at a concentration between 300 and 500 g/L, preferably a concentration of 400 g/L. The Ni2+ ions are added to the electrolyte solution from a soluble salt of Nickel sulfamate tetrahydrate (Ni(SO3NH2)2•4H2O) which is commercially available.
- An acid containing phosphorus, this acid works to provide phosphorus ions (P3+), such that the phosphorus ions react with Ni2+ during the reduction reaction and are co-deposited, thereby forming the amorphous nickel-phosphorus (Ni-P) alloy. Its concentration is of about 0.01 to 30.0 g/L, preferably a concentration of 10.0 g/L.
- A non-toxic, inorganic antibacterial agent, biocompatible with the environment through its whole life-time. The antibacterial agent used does not affect the aesthetic finishing of the electroplated metal coating. The antibacterial agents considered to be used in the formulation of the present invention include antibacterial metals such as: silver or copper nano-particles, having a size between 10 and 100 nanometers, at a concentration of between 3.0 and 10 g/L, the most adequate concentration depends on the current density applied to the coating formation. The occlusion in the metallic matrix of the antibacterial agent nano-particles, provides the coating with the antibacterial feature. Silver or copper nano-particles are commercially available.
- A buffering agent to control the solution pH. This buffering agent is boric acid (H3BO3) and its concentration is of about 8.5 to 45.0 g/L, preferably a 9.5 g/L concentration.
- A cationic surfactant agent having as main function to form a stable suspension with the antibacterial metal nano-particles. Such cationic surfactant provides the particles with a positive charge, which makes easier to electrostatically attract these nano-particles to the cathode surface during the electroplating process, which favors the nano-particles occlusion in the metal matrix, thus producing homogeneous coatings in composition (see Fig. 1). The used surfactant agent does not affect the aesthetic finishing of the electroplated metal composite. The surfactant agent taken into consideration is cationic-type, and it may be cetyl trimethyl ammonium hydrogensulfate, decyl trymethyl ammonium bromide, or sodium dodecyl sulfate. The concentration is between 0.09 and 3.81 g/L, preferably a concentration of 0.2 g/L.
- Either 5% v Hydrochloric acid (HCl) or 5% v sodium hydroxide (NaOH) aqueous solutions, in order to adjust the final pH to between 2 and 5, preferably with a final pH of 3.0.

The electrolytic bath is controlled at a temperature between 30 and 50 °C; particularly satisfactory results are obtained at 40 °C.

The electrolytic bath can be operated in a range of current densities of 0.01 to 0.05 A/cm2. The optimal current density for the bath operation depends on the MANPs concentration used.

The electroplating duration may vary depending on the bath composition, on the current density used and the desired thickness of the coating.

The metallic substrate to be coated can be cathodically electrified using a power source and Nickel soluble anodes.

The bath and method of the present invention are characterized by their versatility, easy control, stability, and they are particularly suitable for obtaining antibacterial metal composite coatings (Ni-P-MANP) in hanging articles in spite of the geometry of the parts to be coated.

### BEST WAY TO PERFORM THE INVENTION

In order to illustrate the composition of the electrolytic bath for electroplating Ni-P-MANPs metal composites in the present invention, the following examples are shown. The examples are provided to illustrate the method and does not represent limiting conditions for the invention.

### Example 1.

An electrolytic suspension (solution A) was prepared having 400 g/L nickel sulfamate tetrahydrate (Ni(SO3NH2)2•4H2O), 9.5 g/L boric acid (H3BO3), 10 g/L Phosphorous acid (H3PO3), 0.2 g/L ammoinum cetyl trimethyl hydrogensulfate, 3.5 g/L silver nano-particles (AgNPs) with an average size of 60 nanometers (nm). The electrolyte pH is adjusted to 3.0 using a 5%v sodium hydroxide (NaOH) solution. The electrolytic suspension was controlled at a temperature of 40°C; an AISI 1018 steel plate was used as the cathode, and a nickel soluble anode, as the anode.

From the previous solution, three Ni-P-AgNPs coatings were formed (coatings 1, 2 and 3) using three different values of current density (A/cm2), using as the cathode, AISI 1018 steel plates, with an exposed area of 15 cm2, according to what following Table 1 shows. The coatings were made in triplicate.

**Table 1.**

| Electrolytic bath operation conditions | | | | |
|---|---|---|---|---|
| Ni-P-AgNPs coating no. | Temperature (°C) | Electrolyte pH | Current Density Applied (A/cm²) | Average Thickness of the Obtained Coating (µm) |
| 1 | 40 | 3.0 | 0.010 | 15 |
| 2 | 40 | 3.0 | 0.016 | 15 |
| 3 | 40 | 3.0 | 0.021 | 15 |

The obtained coatings were adhesive and brightful. The silver concentration in the coatings was determined by using the analysis technique known as inductively coupled plasma spectroscopy "ICP", and the obtained results are shown in Table 2.

The microbiological analysis was performed according to the Mexican Official Standard NOM-109-SSA1-1994, initially and for contact times of 30, 60 and 120 minutes (min) between the Ni-P-AgNPs coating and the solutions contaminated with Escherichia coli and Staphylococcus aureus. Table 2 shows the obtained results in Colony Forming Units (CFU).

**Table 2.**

| Results of AgNPs concentration and of the antibacterial effect in the final coatings | | | | | | | | | |
|---|---|---|---|---|---|---|---|---|---|
| Ni-P-AgNPs Coating No. | AgNPs Concentration (mg/cm³) in the Coating | Staphylococcus aureus bacteria (CFU)Amount by contact time | | | | Escherichia coli bacteria (CFU) amount by contact time | | | |
| | | Initial 0 min | 30 min | 60 min | 120 min | Initial 0 min | 30 min | 60 min | 120 min |
| 1 | 2.06 | 64 | 3 | 2 | 1 | 159 | 5 | 3 | 1 |
| 2 | 1.54 | 64 | 5 | 5 | 1 | 159 | 15 | 5 | 1 |
| 3 | 1.86 | 64 | 18 | 3 | 2 | 159 | 13 | 2 | 3 |

### Example 2.

An electrolytic suspension (solution A) was prepared having 400 g/L nickel sulfamate tetrahydrate (Ni(SO3NH2)2•4H2O), 9.5 g/L boric acid (H3BO3), 10 g/L Phosphorous acid (H3PO3), 0.2 g/L ammoinum cetyl trimethyl hydrogensulfate, 7.0 g/L silver nano-particles (AgNPs) with an average size of 60 nanometers (nm). The electrolyte pH is adjusted to 3.0 using a 5%v sodium hydroxide (NaOH) solution. The electrolytic suspension was controlled at a temperature of 40 °C; an AISI 1018 steel plate was used as the cathode, and a nickel soluble anode, as the anode.

From the previous solution, two Ni-P-AgNPs coatings were formed (coatings 4 and 5) by applying two different values of current density (A/cm2), using as the cathode, AISI 1018 steel plates with an exposed area of 15 cm2, according what Table 3 shows. The coatings were made in triplicate.

**Table 3.**

| Operation conditions of the electrolytic bath | | | | |
|---|---|---|---|---|
| Ni-P-AgNPs Coating No. | Temperature (°C) | Electrolyte pH | Applied Current Density (A/cm²) | obtained Average Coating Thickness (µm) |
| 4 | 40 | 3.0 | 0.013 | 15 |
| 5 | 40 | 3.0 | 0.021 | 15 |

The obtained coatings were adhesive and brightful. The silver concentration in the coatings was determined by using the analysis technique known as inductively coupled plasma spectroscopy "ICP", and the obtained results are shown in Table 4.

The microbiological analysis was performed according to the Mexican Official Standard NOM-109-SSA1-1994, initially and for contact times of 30, 60 and 120 minutes (min) between the Ni-P-AgNPs coating and the solutions contaminated with Escherichia coli and Staphylococcus aureus. Table 4 shows the obtained results in Colony Forming Units (CFU).

**Table 4.**

| Results of AgNPs concentration and of the antibacterial effect in the final coatings | | | | | | | | | |
|---|---|---|---|---|---|---|---|---|---|
| Ni-P-AgNPs Coating No. | AgNPs Concentration (mg/cm³) in the Coating | Staphylococcus aureus bacteria amount (CFU) by contact time | | | | Escherichia coli bacteria amount (CFU) by contact time | | | |
| | | Initial 0 min | 30 min | 60 min | 120 min | Initial 0 min | 30 min | 60 min | 120 min |
| 4 | 2.43 | 64 | 13 | 3 | 3 | 159 | 7 | 1 | 0 |
| 5 | 2.89 | 64 | 7 | 4 | 2 | 159 | 12 | 1 | 1 |

### Example 3.

An electrolytic suspension (solution A) was prepared having 400 g/L nickel sulfamate tetrahydrate (Ni(SO3NH2)2•4H2O), 9.5 g/L boric acid (H3BO3), 10 g/L Phosphorous acid (H3PO3), 0.2 g/L ammoinum cetyl trimethyl hydrogensulfate, 10 g/L silver nano-particles (AgNPs) with an average size of 60 nanometers (nm). The pH of the electrolyte is adjusted to 3.0 using a 5%v sodium hydroxide (NaOH) solution. The electrolytic suspension was controlled at a temperature of 40 °C; an AISI 1018 steel plate was used as the cathode, and a nickel soluble anode, as the anode.

From the previous solution, two Ni-P-AgNPs coatings were formed (coatings 6 and 7) by applying two different values of current density (A/cm2), using as the cathode, AISI 1018 steel plates, with an exposed area of 15 cm2, according to that shown in Table 5. The coatings were made in triplicate.

**Table 5.**

| Operation conditions of the electrolytic bath | | | | |
|---|---|---|---|---|
| Ni-P-AgNPs Coating No. | Temperature (°C) | Electrolyte pH | Applied Current Density (A/cm²) | obtained Coating Average Thickness (µm) |
| 6 | 40 | 3.0 | 0.013 | 15 |
| 7 | 40 | 3.0 | 0.016 | 15 |

The obtained coatings were adhesive and brightful. The silver concentration in the coatings was determined by using the analysis technique known as inductively coupled plasma spectroscopy "ICP", and the results obtained are shown in Table 6.

The microbiological analysis was performed according to the Mexican Official Standard NOM-109-SSA1-1994, initially and for contact times of 30, 60 and 120 minutes (min) between the Ni-P-AgNPs coating and the solutions contaminated with Escherichia coli and Staphylococcus aureus. Table 6 shows the obtained results in Colony Forming Units (CFU).

**Table 6.**

| Results of AgNPs concentration and of the antibacterial effect in the final coatings | | | | | | | | | |
|---|---|---|---|---|---|---|---|---|---|
| Ni-P-AgNPs Coating No. | AgNPs Concentration (mg/cm³) in the Coating | Staphylococcus aureus bacteria amount (CFU) by contact time | | | | Escherichia coli bacteria amount (CFU) by contact time | | | |
| | | Initial 0 min | 30 min | 60 min | 120 min | Initial 0 min | 30 min | 60 min | 120 min |
| 6 | 3.33 | 64 | 1 | 0 | 2 | 159 | 3 | 3 | 2 |
| 7 | 2.83 | 64 | 7 | 1 | 2 | 159 | 3 | 0 | 2 |

In view of all the above-mentioned, the present invention proposes the composition of an electrolytic bath, which by means of the electroplating process, can be applied over electrified metallic substrates in order to obtain a Ni-P-MANPs composite metal coating having homogeneous composition in the whole coating thickness, and with the ability of preventing or inhibiting bacteria growing and/or to eliminate both bacteria types: Gram-negative, such as Escherichia coli, and Gram positive, such as Staphylococcus aureus, at least in 99% of its surface.

The present invention has been described enough such that a person with ordinary skills in the art is able to reproduce it and to obtain the results that are mentioned in the present invention. However, any person skilled in the technical field related to the present invention can be able to make non-described modifications in this application, however, if for the implementation of said modifications in composition the matter claimed in the following claims is required, such compositions should be encompassed within the scope of the present invention.

Having described the invention, it is considered a novelty, and therefore it is claimed as property, the contents in the following claims:

## Claims

1. The composition of the electrolytic bath to electroplate a metal composite of Nickel-phosphorus-metal nano-particles of an antibacterial metal (Ni-P-MANPs), of the type commonly comprising the source of ions to be deposited and salts to make conductive the electrolytic bath, wherein said ion source to be deposited are nickel sulfamate tetrahydrate salts (Ni(SO3NH2)2•4H2O), Phosphorous acid (H3PO3) and antibacterial metal nano-particles, having as additives a buffering pH agent and a surfactant agent, the electrolytic bath has a pH between 2 and 5.

2. The composition of the electrolytic bath to electroplate the metal composite of Nickel-phosphorus-metal nano-particles of an antibacterial metal (Ni-P-MANPs), as claimed in the previous claim, wherein the source of Ni2+ ions is the nickel sulfamate tetrahydrate (Ni(SO3NH2)2•4H2O) at a concentration of 300 to 500 g/L, preferably a concentration of 400 g/L.

3. The composition of the electrolytic bath to electroplate a metal composite of Nickel-phosphorus-metal nano-particles of an antibacterial metal (Ni-P-MANPs), as claimed in claims 1 and 2, wherein the phosphorous acid (H3PO3) is at a concentration of between 0.01 to 30.0 g/L, preferably a concentration of 10.0 g/L.

4. The composition of the electrolytic bath to electroplate a metal composite of Nickel-phosphorus-metal nano-particles of an antibacterial metal (Ni-P-MANPs) as claimed in claims 1 to 3, wherein the antibacterial metal may be silver or copper nano-particles in a concentration between 3.0 and 10 g/L.

5. The composition of the electrolytic bath to electroplate a metal composite of Nickel-phosphorus-metal nano-particles of an antibacterial metal (Ni-P-MANPs) as claimed in claims 1 to 4, wherein the size of the antibacterial metal nano-particles is between 10 to 100 nanometers, preferably a size of 60 nanometers.

6. The composition of the electrolytic bath to electroplate a metal composite of Nickel-phosphorus-metal nano-particles of an antibacterial metal (Ni-P-MANPs), as claimed in claims 1 to 5, wherein the pH buffering agent is boric acid (H3BO3).

7. The composition of the electrolytic bath to electroplate a metal composite of Nickel-phosphorus-metal nano-particles of an antibacterial metal (Ni-P-MANPs) as claimed in claims 1 to 6, wherein the boric acid (H3BO3) is at a concentration of between 8.5 to 45.0 g/L, preferably a concentration of 9.5 g/L.

8. The composition of the electrolytic bath to electroplate a metal composite of Nickel-phosphorus-metal nano-particles of an antibacterial metal (Ni-P-MANPs) as claimed in claims 1 to 7, wherein the surfactant considered is that of the cationic type, and it may be ammonium cetyl trimethyl hydrogensulfate, decyl trymethyl ammonium bromide, or sodium dodecyl sulfate.

9. The composition of the electrolytic bath to electroplate a metal composite of Nickel-phosphorus-metal nano-particles of an antibacterial metal (Ni-P-MANPs) as claimed in claims 1 to 8, wherein the surfactant agent is at a concentration between 0.09 and 3.81 g/L, preferably a concentration of 0.2 g/L.
